# EUROPÄISCHE PATENTANMELDUNG

(11) **EP 4 616 937 A1**
(43) Veröffentlichungstag der Anmeldung: **17.09.2025**
(21) Anmeldenummer: 24163712.3
(22) Anmeldetag: 15.03.2024
(51) Int. Cl.: B01F 31/441, B01F 33/501, B01F 35/71, B01F 101/20

(54) **KNOCHENZEMENT-MISCHSYSTEM MIT EINSTICHVERBINDUNG UND MULTIFUNKTIONSBEDIENUNG**

(71) Anmelder: Heraeus Medical GmbH, 61273 Wehrheim (DE)
(72) Erfinder: Dr. Vogt, Sebastian, 61273 Wehrheim (DE); Dr. Kluge, Thomas, 61273 Wehrheim (DE)
(74) Vertreter: Heraeus IP

(57) **Zusammenfassung**

Die vorliegende Erfindung betrifft eine Vorrichtung zur Herstellung von Knochenzement, aufweisend ein Basisteil (100), welches einen Behälter (101) mit einem Träger (118) zur Aufnahme eines Gefäßes mit Monomerflüssigkeit, ein Bodenteil (103) mit einem Sammelbereich (104), einen Hohldorn (105) mit einer Spitze (106), und einen Kolben (107) mit einer Durchführung (108) zur Aufnahme des Hohldorns (105) aufweist, und eine Kartusche (200) zur Aufnahme eines PMMA-Knochenzementpulvers (201), wobei die Kartusche ein Septum (202) aufweist, welches die Kartusche nach außen begrenzt; wobei die Vorrichtung weiterhin ein Bedienelement (124) aufweist, und wobei das Bedienelement (124) eingerichtet ist, ein Gefäß mit Monomerflüssigkeit zu öffnen und nachfolgend eine aus dem Gefäß freigesetzte Monomerflüssigkeit durch Bewegung des Kolbens aus dem Sammelbereich durch den Hohldorn in die Kartusche zu pumpen.

## Beschreibung

### GEBIET DER ERFINDUNG

Die vorliegende Erfindung betrifft das Gebiet der Medizintechnik, insbesondere Vorrichtungen zur Herstellung eines PMMA-Knochenzements und zur Lagerung von Komponenten davon.

### STAND DER TECHNIK

Polymethylmethacrylat-Knochenzemente gehen auf die grundlegenden Arbeiten von CHARNLEY zurück. Polymethylmethacrylat-Knochenzemente bestehen üblicherweise aus einer flüssigen Monomerkomponente und einer Pulverkomponente. Die Monomerkomponente kann beispielsweise das Monomer Methylmethacrylat und einen darin gelösten Aktivator (z.B. N,N-Dimethyl-p-toluidin) enthalten. Die Pulverkomponente, auch als Knochenzementpulver bezeichnet, kann ein oder mehrere Polymere aufweisen, die auf Basis von Methylmethacrylat und Comonomeren, wie Styren, Methylacrylat oder ähnlichen Monomeren durch Polymerisation, vorzugsweise Suspensionspolymerisation, herstellbar sein können. Die Pulverkomponente kann weiterhin einen Röntgenopaker und einen Initiator wie z.B. Dibenzoylperoxid aufweisen. Beim Vermischen der Pulverkomponente mit der Monomerkomponente kann durch Quellung der Polymere der Pulverkomponente im Methylmethacrylat ein plastisch verformbarer Teig entstehen, der eigentliche Knochenzementteig. Beim Vermischen der Pulverkomponente mit der Monomerkomponente reagiert beispielsweise der Aktivator N,N-Dimethyl-p-toluidin mit Dibenzoylperoxid unter Bildung von Radikalen. Die gebildeten Radikale können die radikalische Polymerisation des Methylmethacrylates initiieren. Mit fortschreitender Polymerisation des Methylmethacrylates erhöht sich die Viskosität des Zementteigs, bis dieser erstarrt. Polymethylmethacrylat-Knochenzemente können in geeigneten Mischbechern mit Hilfe von Spateln durch Vermischen der Pulverkomponente mit der Monomerflüssigkeit vermischt werden. Dabei kann es zum Einschluss von Luftblasen im Knochenzementteig kommen, welche die mechanischen Eigenschaften des ausgehärteten Knochenzements negativ beeinflussen können.

Zur Vermeidung von Lufteinschlüssen im Knochenzementteig wurden eine Vielzahl von Vakuum-Zementiersystemen beschrieben, von denen exemplarisch folgende genannt sind: US 6,033,105 A, US 5,624,184 A, US 4,671,263 A, US 4,973,168 A, US 5,100,241 A, WO 99/67015 A1, EP 1 020 167 A2, US 5,586,821 A, EP 1 016 452 A2, DE 36 40 279 A1, WO 94/26403 A1, EP 1 005 901 A2, US 5,344,232 A.

Eine Weiterentwicklung in der Zementiertechnik stellen Zementiersysteme dar, in denen sowohl das Zementpulver als auch die Monomerflüssigkeit bereits in separaten Kompartimenten der Mischsysteme verpackt sind und die erst unmittelbar vor der Zementapplikation im Zementiersystem miteinander vermischt werden. Solche geschlossenen "Full-Prepacked-Mischsysteme" sind in den Patentdokumenten EP0380867B1, EP0796653B1, EP0692229B1, US5997544A, US6709149B1, DE69812726T2 und US5588745A, WO9416951A1, DE19718648A1, EP1741413B1, EP3054880B1, DE102009031178B3, US8662736B2, EP2269718B1, EP2281532B1 und EP3093067B1 beschrieben.

Bei Full-Prepacked-Mischsystemen stellen sich einige Herausforderungen. Wünschenswert ist der sichere, reproduzierbare Transfer der Monomerflüssigkeit in das Polymethylmethacrylat-Knochenzementpulver und die Vermeidung einer unkontrollierten Mischung der Komponenten während der Lagerung. Vorteilhaft ist, wenn das Zementpulver während des Transports und der Lagerung des Mischsystems nicht in das Leitungsmittel zum Transfer der Monomerflüssigkeit eindringen kann. Ansonsten kann das Leitungsmittel beim Monomertransfer durch Quellung und Verklebung verstopfen. Eine Blockierung des Leitungsmittels durch gequollenen, klebenden Knochenzement könnte den Monomerflüssigkeitstransfer unterbrechen und die Vermischung der Zementkomponenten im vorbestimmten Mischungsverhältnis verhindern.

Bei den Full-Prepacked-Mischsystemen gemäß EP0796653B1, US6709149B1, EP1741413B1, EP3054880B1, EP2269718B1 und EP2281532B1 erfolgt der Monomertransfer durch Anlegen eines externen Vakuums an die Kartusche über einen Vakuumanschluss und einer porösen Platte. Das bedeutet, dass die Monomerflüssigkeit durch das angelegte Vakuum in die Kartusche gesaugt wird. Bei dem in der EP3093067B1 beschriebenen Mischsystem kann entweder ein Transfer der Monomerflüssigkeit in die Kartusche durch Vakuumeinwirkung, oder alternativ ein Drucktransfer der Monomerflüssigkeit durch einen in das Mischsystem integrierten, manuell bedienbaren Pumpkolben erfolgen.

Die Blockierung des Leitungsmittels zum Transfer der Monomerflüssigkeit wird gemäß EP2281532B1 dadurch vermieden, dass ein für die Monomerflüssigkeit permeables Netz oder eine Porenscheibe zum Blockieren der Zementpulverpartikel genutzt wird, wobei das Netz oder die Porenscheibe Bestandteil einer speziellen Düse sind, die einen scharfen Monomerflüssigkeitsstrahl beim Transfer erzeugt, der gequollene Zementpartikel verdrängt, so dass ein vollständiger Monomerflüssigkeitstransfer in das Knochenzementpulver möglich ist. EP1741413B1 offenbart ein Verfahren zum Inkontaktbringen eines Pulvers und einer flüssigen Komponente, wobei ein Flüssigkeitsbehälter durch Eindringen einer Kanüle in einen Wandabschnitt des Flüssigkeitsbehälters geöffnet wird, der ein seitliches Loch in einem inneren Rohrelement im Flüssigkeitsbehälter verschließt.

In der Patentschrift EP3636338B1 ist ein Mischsystem offenbart, das einen Verbindungszylinder aufweist, der dazu konfiguriert ist, selektiv an eine Mischkammer gekoppelt und von dieser entfernbar zu sein, und wobei ein Ventil dazu konfiguriert ist, die Verbindung zwischen dem Verbindungszylinder und dem Ventil abzudichten, wenn sie selektiv miteinander gekoppelt sind, und dazu konfiguriert ist, die Mischkammer abzudichten, wenn der Verbindungszylinder aus der Mischkammer entnommen wird. Die EP3490700B1 offenbart ein ähnliches Mischsystem.

### BEVORZUGTE AUSFÜHRUNGSFORMEN

Eine Aufgabe der vorliegenden Erfindung besteht darin, eines oder mehrere der oben geschilderten und weitere Probleme des Stands der Technik zu lösen. Beispielsweise liefert die Erfindung eine Vorrichtung zum Lagern und Mischen von Knochenzement, die eine einfache und zuverlässige Mischung einer Monomerflüssigkeit mit einer Pulverkomponente eines PMMA-Knochenzements erlaubt, um einen gebrauchsfertigen PMMA-Knochenzementteig daraus herzustellen. Die erfindungsgemäßen Vorrichtungen können mehrere Teile aufweisen, die in einfacher Weise miteinander verbunden und wieder voneinander getrennt werden können, und dabei eine zuverlässige und sichere Überführung einer Monomerflüssigkeit von einem Teil der Vorrichtung in das andere Teil der Vorrichtung erlauben. Ein Austreten von Monomerflüssigkeit oder Eindringen von Kontaminationen können durch ein selbstdichtendes Verbindungssystem vermieden werden. Die erfindungsgemäße Vorrichtung kann eine einfache Handhabung bieten. Eine Monomerflüssigkeit und eine Pulverkomponente eines PMMA-Knochenzements können in der Vorrichtung getrennt voneinander gelagert, kontrolliert zusammengeführt und miteinander gemischt werden. Die Vorrichtung kann in einem Innenraum der Vorrichtung eine Monomerflüssigkeit aus einem geschlossenen Gefäß freisetzen und diese mithilfe eines beweglichen Kolbens in einen anderen Teil der Vorrichtung überführen. Die Vorrichtung kann diese Funktionen ausüben, während sie sicher auf einer Unterlage aufgestellt ist. Die Monomerflüssigkeit kann vollständig überführt werden, wobei eine Verstopfung der Vorrichtung durch verklumptes Pulver vermieden werden kann.

Diese Aufgaben werden gelöst durch die hierin beschriebenen Verfahren, Vorrichtungen, Kits und medizinischen Verwendungen, insbesondere denjenigen, die in den Patentansprüchen beschrieben sind.

Bevorzugte Ausführungsformen der Erfindung werden nachfolgend beschrieben.

Eine erste Ausführungsform betrifft eine Vorrichtung zur Herstellung von Knochenzement, aufweisend ein Basisteil, welches einen Behälter mit einem Träger zur Aufnahme eines Gefäßes mit Monomerflüssigkeit, ein Bodenteil mit einem Sammelbereich, einen Hohldorn mit einer Spitze, und einen Kolben mit einer Durchführung zur Aufnahme des Hohldorns aufweist, und eine Kartusche zur Aufnahme eines PMMA-Knochenzementpulvers, wobei die Kartusche ein Septum aufweist, welches die Kartusche nach außen begrenzt;
wobei die Vorrichtung weiterhin ein Bedienelement aufweist, und wobei das Bedienelement eingerichtet ist, ein Gefäß mit Monomerflüssigkeit zu öffnen und nachfolgend eine aus dem Gefäß freigesetzte Monomerflüssigkeit durch Bewegung des Kolbens aus dem Sammelbereich durch den Hohldorn in die Kartusche zu pumpen.

Eine zweite Ausführungsform betrifft eine Vorrichtung gemäß der ersten Ausführungsform, wobei das Bedienelement beweglich an dem Basisteil angeordnet ist.

Eine dritte Ausführungsform betrifft eine Vorrichtung gemäß der ersten oder zweiten Ausführungsform, wobei das Bedienelement entlang einer Achse verschiebbar und bevorzugt um diese Achse schwenkbar ist.

Eine vierte Ausführungsform betrifft eine Vorrichtung gemäß einer der vorangehenden Ausführungsformen, wobei das Bedienelement in einer ersten Anordnung eingerichtet ist, eine Kraft auf ein Gefäß mit Monomerflüssigkeit auszuüben, und dabei ein vollständiges Einschieben des Kolbens in den Sammelbereich zu verhindern.

Eine fünfte Ausführungsform betrifft eine Vorrichtung gemäß einer der vorangehenden Ausführungsformen, wobei das Bedienelement in einer zweiten Anordnung eingerichtet ist, ein Einschieben des Kolbens in den Sammelbereich zu ermöglichen, um eine Monomerflüssigkeit aus dem Sammelbereich durch den Hohldorn in die Kartusche zu pumpen.

Eine sechste Ausführungsform betrifft eine Vorrichtung gemäß einer der vorangehenden Ausführungsformen, weiterhin aufweisend ein Öffnungselement, wobei das Bedienelement operativ mit dem Öffnungselement verbindbar oder verbunden ist, wobei das Öffnungselement zum Öffnen eines Gefäßes mit Monomerflüssigkeit ausgebildet und eingerichtet ist.

Eine siebte Ausführungsform betrifft eine Vorrichtung gemäß einer der vorangehenden Ausführungsformen, wobei das Bedienelement operativ mit dem Kolben verbindbar oder verbunden ist.

Eine achte Ausführungsform betrifft eine Vorrichtung gemäß der siebten Ausführungsform, wobei das Bedienelement ein Drehgelenk aufweist.

Eine neunte Ausführungsform betrifft eine Vorrichtung gemäß einer der vorangehenden Ausführungsformen, wobei die Vorrichtung ausgebildet und eingerichtet ist, mittels Durchstechen des Septums mit dem Hohldorn eine fluidleitende Verbindung zwischen dem Basisteil und der Kartusche zu bilden, um eine Monomerflüssigkeit aus dem Sammelbereich in die Kartusche zu überführen.

Eine zehnte Ausführungsform betrifft eine Vorrichtung gemäß einer der vorangehenden Ausführungsformen, wobei die Kartusche lösbar mit dem Basisteil verbindbar ist.

Eine elfte Ausführungsform betrifft eine Vorrichtung gemäß einer der vorangehenden Ausführungsformen, wobei der Kolben durch Einschieben der Kartusche in das Basisteil beweglich ist.

Eine zwölfte Ausführungsform betrifft eine Vorrichtung gemäß einer der vorangehenden Ausführungsformen, wobei das Basisteil einen Anschlag aufweist, um die Bewegung des Kolbens in dem Basisteil zu begrenzen, wobei das Bedienelement bevorzugt eingerichtet ist, in den Anschlag einzugreifen.

Eine dreizehnte Ausführungsform betrifft eine Vorrichtung gemäß einer vorangehenden Ausführungsformen, wobei das Basisteil einen Verbindungskanal mit einer Ausgangsöffnung aufweist, wobei der Verbindungskanal eine fluidleitende Verbindung zwischen dem Behälter und dem Sammelbereich bildet.

Eine vierzehnte Ausführungsform betrifft eine Vorrichtung gemäß der dreizehnten Ausführungsform, wobei der Kolben ausgebildet und eingerichtet ist, die Ausgangsöffnung des Verbindungskanals flüssigkeitsdicht zu verschließen, wenn der Kolben in das Basisteil eingeschoben wird.

Eine fünfzehnte Ausführungsform betrifft eine Vorrichtung gemäß einer der vorangehenden Ausführungsformen, wobei die Vorrichtung ausgestaltet und eingerichtet ist, eine Monomerflüssigkeit aus dem Basisteil in die Kartusche zu überführen, während das Basisteil auf einer Unterlage aufgestellt ist, so dass das Bodenteil in Richtung der Unterlage ausgerichtet ist.

### KURZE BESCHREIBUNG DER ZEICHNUNGEN

**Figur 1** zeigt den Querschnitt des Basisteils einer erfindungsgemäßen Vorrichtung.
**Figur 2** zeigt den Querschnitt einer erfindungsgemäßen Vorrichtung, wobei ein Basisteil mit einer Kartusche in Kontakt gebracht ist.
**Figur 3** zeigt den Querschnitt einer erfindungsgemäßen Vorrichtung, wobei eine Kartusche auf ein Basisteil aufgesetzt ist, um ein Septum der Kartusche mit einem Hohldorn des Basisteils zu durchstechen.
**Figur 4** zeigt den Querschnitt einer erfindungsgemäßen Vorrichtung, wobei das Bedienelement eine Kraft auf das Öffnungselement ausübt, sodass eine Glasampulle in dem Behälter geöffnet wird.
**Figur 5** zeigt den Querschnitt einer erfindungsgemäßen Vorrichtung, wobei eine Kartusche auf ein Basisteil aufgesetzt ist, wobei das Septum der Kartusche mit einem Hohldorn des Basisteils durchstochen ist.
**Figur 6** zeigt den Querschnitt einer erfindungsgemäßen Vorrichtung, wobei ein Kolben in das Basisteil eingeschoben ist, um eine Ausgangsöffnung eines Verbindungskanals flüssigkeitsdicht zu verschließen.
**Figur 7** zeigt einen Querschnitt einer Kartusche mit einem Mischstab zum Mischen einer Monomerflüssigkeit mit einem PMMA-Knochenzementpulver in einem Innenraum der Kartusche.
**Figur 8** zeigt einen Querschnitt einer Kartusche mit einer Gewindestange und einem Austragsrohr zur Abgabe von Knochenzementteig aus der Kartusche.

### AUSFÜHRLICHE BESCHREIBUNG

Zu den hierin beschriebenen Ausführungsformen, deren Elemente ein bestimmtes Merkmal (z.B. ein Material) "aufweisen", "enthalten", oder "umfassen" wird grundsätzlich immer eine weitere Ausführungsform erwogen, in denen das betreffende Element allein aus dem Merkmal besteht, d.h. keine weiteren Bestandteile umfasst. Das Wort "umfassen" oder "umfassend" wird hierin synonym mit dem Wort "enthalten", "enthaltend", "aufweisen" oder "aufweisend" verwendet.

"Operativ verbunden" oder "operativ verbindbar", bedeutet hierin, dass zwei betreffende Elemente eine funktionelle Beziehung zueinander aufweisen. Beispielsweise kann ein erstes Element eingerichtet sein, ein zweites Element durch eine solche operative Verbindung zu steuern oder zu bewegen. Der Begriff "steuern" umfasst hierin auch die Sperrung oder Freigabe einer Funktion, beispielsweise das Ermöglichen oder die Einschränkung der Bewegung oder sonstigen Funktion eines Elements.

Wenn in einer Ausführungsform ein Element mit dem Singular bezeichnet ist, wird ebenfalls eine Ausführungsform erwogen, bei denen mehrere dieser Elemente vorhanden sind. Die Verwendung eines Begriffs für ein Element im Plural umfasst grundsätzlich auch eine Ausführungsform, in welchem nur ein einzelnes entsprechendes Element enthalten ist.

Soweit nicht anders angegeben oder aus dem Zusammenhang eindeutig ausgeschlossen, ist es grundsätzlich möglich und wird hiermit eindeutig in Betracht gezogen, dass Merkmale unterschiedlicher Ausführungsformen auch in den anderen hierin beschriebenen Ausführungsformen vorhanden sein können. Ebenso wird grundsätzlich erwogen, dass alle Merkmale, die hierin in Zusammenhang mit einem Verfahren beschrieben werden, auch für die hierin beschriebenen Erzeugnisse, Vorrichtungen, Kits und Verwendungen anwendbar sind, und umgekehrt. Lediglich aus Gründen der knapperen Darstellung werden alle diese erwogenen Kombinationen nicht in allen Fällen explizit aufgeführt. Auch technische Lösungen, die zu den hierin beschriebenen Merkmalen bekanntermaßen gleichwertig sind, sollen grundsätzlich vom Umfang der Erfindung umfasst sein.

Eine erste Ausführungsform betrifft eine Vorrichtung zur Herstellung von Knochenzement, aufweisend ein Basisteil, welches einen Behälter mit einem Träger zur Aufnahme eines Gefäßes mit Monomerflüssigkeit, ein Bodenteil mit einem Sammelbereich, einen Hohldorn mit einer Spitze, und einen Kolben mit einer Durchführung zur Aufnahme des Hohldorns aufweist, und eine Kartusche zur Aufnahme eines PMMA-Knochenzementpulvers, wobei die Kartusche ein Septum aufweist, welches die Kartusche nach außen begrenzt;
wobei die Vorrichtung weiterhin ein Bedienelement aufweist, und wobei das Bedienelement eingerichtet ist, ein Gefäß mit Monomerflüssigkeit zu öffnen und nachfolgend eine aus dem Gefäß freigesetzte Monomerflüssigkeit durch Bewegung des Kolbens aus dem Sammelbereich durch den Hohldorn in die Kartusche zu pumpen.

Die erfindungsgemäße Vorrichtung kann zwei miteinander verbindbare Teile aufweisen, nämlich ein Basisteil und eine Kartusche. Das Basisteil weist einen Behälter zur Aufnahme einer Monomerflüssigkeit auf. Der Behälter weist einen Träger zur Aufnahme eines Gefäßes mit Monomerflüssigkeit, zum Beispiel einer Glasampulle oder eines Beutels, auf. Solche Glasampullen oder Beutel werden als handelsübliche Verpackungen für Monomerflüssigkeit eines PMMA-Knochenzements verwendet. Der Behälter kann mit Monomerflüssigkeit, zum Beispiel gasdicht verpackt in einer Glasampulle oder einem Beutel, vorbefüllt sein. Alternativ kann der Behälter leer bereitgestellt und vom Benutzer mit Monomerflüssigkeit befüllbar sein, beispielsweise durch Einbringen einer Glasampulle mit Monomerflüssigkeit in den Behälter. Ebenso kann die Kartusche leer oder mit PMMA-Knochenzementpulver vorbefüllt bereitgestellt werden. Das Basisteil kann einen transparenten Bereich aufweisen. Beispielsweise kann der Behälter einen transparenten Bereich aufweisen. Der Sammelbereich kann ebenfalls einen transparenten Bereich aufweisen. Dies kann es einem Benutzer der Vorrichtung erlauben, das vollständige Abfließen von Monomerflüssigkeit aus dem Behälter in den Sammelbereich von außen visuell zu überprüfen. Weiterhin kann das Basisteil eine Füllstandsmarkierung aufweisen. Eine solche Füllstandsmarkierung kann beispielsweise an der Position eines gewünschten Flüssigkeitsspiegels des Sammelbereichs angeordnet sein, welcher durch eine vollständige Überführung einer Monomerflüssigkeit aus dem Behälter in den Sammelbereich erzielbar ist.

Die Vorrichtung weist ein Bedienelement auf. Das Bedienelement ist eingerichtet, ein Gefäß mit Monomerflüssigkeit zu öffnen. Beispielsweise kann das Bedienelement eingerichtet sein, unmittelbar oder indirekt eine Kraft auf ein Gefäß mit Monomerflüssigkeit auszuüben, um dieses aufzubrechen und eine Monomerflüssigkeit aus dem Gefäß freizusetzen. Das Bedienelement ist bevorzugt eingerichtet, ein Gefäß mit Monomerflüssigkeit innerhalb des Behälters der Vorrichtung zu öffnen.

Das Bedienelement ist eingerichtet, eine aus dem Gefäß freigesetzte Monomerflüssigkeit durch Bewegung des Kolbens aus dem Sammelbereich durch den Hohldorn in die Kartusche zu pumpen. Dies bedeutet, dass abhängig von einer Anordnung oder sonstigen Konfiguration des Bedienelements eine Pumpfunktion der Vorrichtung ermöglicht wird, welche eine Monomerflüssigkeit aus dem Sammelbereich in die Kartusche überführt. Das Bedienelement kann demnach beispielsweise die Pumpfunktion des Kolbens ermöglichen, indem es eine Sperrung des Kolbens freigibt. Alternativ oder zusätzlich kann das Bedienelement eine Kraft auf den Kolben ausüben, um die Pumpfunktion des Kolbens zu betreiben.

Das Bedienelement ist demzufolge eingerichtet, ein Gefäß mit Monomerflüssigkeit zu öffnen und nachfolgend eine aus dem Gefäß freigesetzte Monomerflüssigkeit durch Bewegung des Kolbens aus dem Sammelbereich durch den Hohldorn in die Kartusche zu pumpen.

Das Bedienelement kann beweglich an dem Basisteil angeordnet sein. Das Bedienelement kann entlang einer Achse verschiebbar sein. Diese Achse kann parallel zu einer Bewegungsrichtung des Kolbens verlaufen. Die Achse kann sich in Richtung eines Bodenteils des Basisteils erstrecken, insbesondere in Richtung einer Aufstandsfläche des Bodenteils. Hierdurch kann das Bedienelement zumindest abschnittsweise in das Basisteil eingeschoben werden. Das Bedienelement kann weiterhin um eine solche Achse schwenkbar sein. Hierdurch kann das Bedienelement von einer ersten Anordnung in eine zweite Anordnung gebracht werden. Beispielsweise kann das Bedienelement hierzu ein Drehgelenk aufweisen. Das Drehgelenk kann beispielsweise als ringförmige Halterung ausgestaltet sein, welche in ein zylindrisches Gehäuseteil des Basisteils eingreift. Das Bedienelement kann einen Griff aufweisen, der zur Bewegung des Bedienelements durch einen Benutzer ausgebildet und eingerichtet ist. Der Griff kann eine flache Oberfläche aufweisen, mit dessen Hilfe ein Benutzer das Bedienelement mit einer Hand, beispielsweise mit einem Finger, teilweise in das Basisteil, oder in Richtung des Basisteils, einschieben kann.

In einer ersten Anordnung kann das Bedienelement eingerichtet sein, eine Kraft auf ein Gefäß mit Monomerflüssigkeit auszuüben, und dabei ein vollständiges Einschieben des Kolbens in den Sammelbereich zu verhindern. Hierdurch kann ein Gefäß mit Monomerflüssigkeit, beispielsweise eine Glasampulle oder ein Folienbeutel, geöffnet werden. Die Monomerflüssigkeit kann dann aus dem geöffneten Gefäß in den Sammelbereich gelangen, bevorzugt durch freien Gravitationsfluss. In der ersten Anordnung ist die Vorrichtung bevorzugt eingerichtet, eine Monomerflüssigkeit in dem Sammelbereich zu halten. In der ersten Anordnung erfolgt bevorzugt keine Überführung der Monomerflüssigkeit in die Kartusche. Dies ermöglicht eine zeitliche Trennung zwischen der Freisetzung der Monomerflüssigkeit aus dem Gefäß einerseits und der Überführung der Monomerflüssigkeit in die Kartusche andererseits. Das Bedienelement kann in einer zweiten Anordnung eingerichtet sein, ein Einschieben des Kolbens in den Sammelbereich zu ermöglichen. Hierdurch kann eine Monomerflüssigkeit aus dem Sammelbereich durch den Hohldorn in die Kartusche gepumpt werden.

Wie oben dargelegt, kann das Bedienelement bevorzugt durch Schwenken um eine Achse von einer ersten Anordnung in eine zweite Anordnung gebracht werden. In gleichartiger Weise sind auch andere technische Lösungen möglich, um das Bedienelement von einer ersten Anordnung in eine zweite Anordnung zu bringen. Hierdurch ist eine Funktionswahl des Bedienelements möglich, wonach das Bedienelement abhängig von der Anordnung des Bedienelements entweder zum Öffnen eines Gefäßes mit Monomerflüssigkeit oder zum Pumpen einer Monomerflüssigkeit aus dem Sammelbereich in die Kartusche eingerichtet ist.

Die Vorrichtung kann weiterhin ein Öffnungselement aufweisen. Das Öffnungselement ist bevorzugt zum Öffnen eines Gefäßes mit Monomerflüssigkeit ausgebildet und eingerichtet. Das Bedienelement ist bevorzugt operativ mit dem Öffnungselement verbindbar, oder damit verbunden. Beispiele für ein Öffnungselement beinhalten einen Stift oder einen Keil, der gegen den Kopf einer Glasampulle gedrückt werden kann, um den Kopf von der Glasampulle abzubrechen. Beispielsweise kann das Bedienelement eingerichtet sein, eine Kraft über das Öffnungselement auf ein Gefäß mit Monomerflüssigkeit zu übertragen, um das Gefäß zu öffnen und Monomerflüssigkeit daraus freizusetzen. Das Bedienelement ist bevorzugt angeordnet, um in das Öffnungselement eingreifen zu können. Das Öffnungselement kann funktionell mit dem Kolben verbunden sein. Der Kolben kann ausgebildet und eingerichtet sein, das Öffnungselement zu bewegen.

Das Öffnungselement ist bevorzugt zur Öffnung eines Folienbeutels oder einer Glasampulle innerhalb der Vorrichtung eingerichtet. Ein Beispiel für ein Öffnungselement zur Öffnung eines Folienbeutels ist ein Dorn, welcher zum Durchstechen eines Folienbeutels geeignet ist. Ein solcher Dorn kann beispielsweise aus einem starren Kunststoff oder aus Metall ausgebildet sein. Ein weiteres Beispiel für ein Öffnungselement zur Öffnung einer Glasampulle ist ein Vorsprung, gegen den der Kopf einer Glasampulle gedrückt werden kann, um sie aufzubrechen. Ein Mittel zur Öffnung einer Glasampulle kann auch ein beweglich angeordnetes Element wie z.B. einen Kolben, aufweisen, welches die Glasampulle gegen ein hartes Gehäuseteil, z.B. einen Vorsprung, drücken und dadurch aufbrechen kann. Öffnungselemente zur Öffnung eines Folienbeutels oder einer Glasampulle sind weiterhin in DE19532015A1, WO9718031A1, EP2404864B1 und WO2010012114A1 offenbart.

In einer Ausführungsform weist der Behälter einen in dem Behälter angeordneten Vorsprung auf, der zum Aufbrechen einer in dem Behälter aufnehmbaren Glasampulle ausgebildet und eingerichtet ist, wobei das Bedienelement ausgebildet und eingerichtet ist, eine in der Halterung aufnehmbare Glasampulle durch Druck der Glasampulle gegen den Vorsprung aufzubrechen.

In einer Ausführungsform weist das Basisteil einen Anschlag auf. Der Anschlag kann ausgebildet und eingerichtet sein, die Bewegung des Kolbens in dem Basisteil zu begrenzen. Das Bedienelement kann eingerichtet sein, in den Anschlag einzugreifen. Insbesondere kann das Bedienelement in einer hierin beschriebenen ersten Anordnung eingerichtet sein, in den Anschlag einzugreifen. Hierdurch kann verhindert werden, dass in einer ersten Anordnung des Bedienelements Monomerflüssigkeit aus dem Sammelbereich in die Kartusche gepumpt wird. Der Anschlag kann beispielsweise an einer Außenseite des Basisteils angeordnet sein. Der Anschlag kann als Vorsprung ausgestaltet sein, welcher sich in Richtung des Bedienelements erstreckt.

Das Basisteil kann zum Aufstellen auf eine Unterlage eingerichtet sein. Das Basisteil weist ein Bodenteil auf, das eine flache, bevorzugt planare, Außenseite aufweisen kann. Das Bodenteil weist einen Sammelbereich auf. Der Sammelbereich ist bevorzugt zur Aufnahme einer Monomerflüssigkeit ausgebildet und eingerichtet. Beispielsweise kann das Basisteil eingerichtet sein, Monomerflüssigkeit aus einem Gefäß, wie z.B. einer Glasampulle oder einem Folienbeutel, freizusetzen, und durch Gravitationsfluss in den Sammelbereich zu überführen. Der Sammelbereich kann eine Vertiefung aufweisen, beispielsweise eine konkave Wölbung, vergleichbar mit einer Uhrglasschale. Der Querschnitt der Vertiefung kann U-förmig oder V-förmig sein. Die Vertiefung kann eine trichterförmige Form aufweisen.

Der Sammelbereich weist bevorzugt eine Aufnahmekapazität für Monomerflüssigkeit auf, welche das Volumen der in dem Behälter enthaltenen oder aufnehmbaren Monomerflüssigkeit übersteigt. Beispielsweise kann der Sammelbereich ein Volumen aufweisen, welches mindestens das 1,1-fache Volumen der in dem Behälter enthaltenen oder aufnehmbaren Monomerflüssigkeit beträgt. Wenn beispielsweise der Behälter einen Träger zur Aufnahme von handelsüblichen Glasampullen mit 10 ml Monomerflüssigkeit aufweist, weist der Sammelbereich bevorzugt ein Volumen von mindestens 11 ml auf. Dies gilt in entsprechender Weise für Ausführungsformen, die einen Träger zur Aufnahme von Glasampullen mit 5 ml, 20 ml, oder 30 ml Monomerflüssigkeit aufweisen.

Das Basisteil weist weiterhin einen Hohldorn mit einer Spitze auf. Der Hohldorn weist bevorzugt einen Innenraum auf, der das Hindurchfließen einer Flüssigkeit durch den Hohldorn erlaubt. Der Innenraum kann einen Durchmesser von 1,0 mm bis 3,0 mm aufweisen. Der Hohldorn kann Auslassöffnungen mit einen Durchmesser kleiner 1,5 mm und besonders bevorzugt kleiner 1,0 mm aufweisen. Durch einen geringen Durchmesser des Hohldorns kann beim Überführen einer Monomerflüssigkeit aus dem Sammelbereich in die Kartusche ein scharfer Strahl ausgebildet werden, welcher gegebenenfalls kleinere Partikel aus Knochenzementpulver verdrängen kann. Auf diese Weise kann eine Verstopfung des Hohldorns vermieden werden. Der Hohldorn kann eine angeschrägte Spitze, ähnlich einer geschliffenen Kanüle, aufweisen, wobei sich bevorzugt in einer Schräge der Spitze eine Auslassöffnung des Hohldorns befindet. Der Hohldorn kann eine runde Spitze aufweisen. Hierbei können nahe der der Spitze ein oder mehrere Öffnungen angeordnet sein, die mit dem Innenraum des Hohldorns verbunden sind. Weiterhin kann der Innenraum des Hohlraums einen Absatz aufweisen, an dem ein poröser, für Flüssigkeiten durchlässiger Stift gestützt ist. Dieser Stift kann als Filter dienen, wie nachfolgend hierin beschrieben. Der Innenraum des Hohldorns kann in Richtung des Absatzes verjüngt sein. Der Absatz kann eingerichtet sein, den Stift in dem Hohldorn zu halten. Der Hohldorn kann aus Kunststoff oder Metall, z.B. Edelstahl, ausgebildet sein. Beispiele für verwendbare Kunststoffe umfassen Polyamid 12, Polyamid 6, Polyamid 66 und Polyethersulfon. Der Hohldorn, insbesondere die Spitze des Hohldorns, kann durch eine abnehmbare Schutzkappe bedeckt sein.

Die Kartusche weist ein Septum auf, welches die Kartusche nach außen begrenzt. Das Septum ist bevorzugt in einem Bereich der Kartusche angeordnet, der zur Verbindung mit dem Basisteil ausgebildet und eingerichtet ist. Das Septum kann in einem Austragskolben oder in einem Kartuschenkopf der Kartusche angeordnet sein. Das Septum ist mit dem Hohldorn des Basisteils durchstechbar. Das Septum kann eine Perforation aufweisen, welche das Durchstechen des Septums an einer vorbestimmten Position des Septums erleichtert. In einer Konfiguration der Vorrichtung, in welcher das Septum mit dem Hohldorn durchstochen ist, kann sich eine fluidleitende Verbindung zwischen dem Basisteil und der Kartusche bilden. Dies ermöglicht es, eine Monomerflüssigkeit aus dem Sammelbereich des Basisteils in die Kartusche zu überführen. Hierbei kann durch die selbstdichtende Funktion des Septums verhindert werden, dass Monomerflüssigkeit aus der Vorrichtung nach außen dringt. Dies gilt auch, wenn der Hohldorn wieder aus dem Septum herausgezogen wird, da sich die Einstichstelle durch die Rückstellkraft des Septums selbsttätig verschließen kann. Weiterhin kann hierdurch verhindert werden, dass Kontaminationen von außen in die Vorrichtung eindringen. In einer Ausführungsform ist die Vorrichtung ausgebildet und eingerichtet, mittels Durchstechen des Septums mit dem Hohldorn eine fluidleitende Verbindung zwischen dem Basisteil und der Kartusche zu bilden, um eine Monomerflüssigkeit aus dem Sammelbereich in die Kartusche zu überführen.

Der Begriff "fluidleitende Verbindung" oder "flüssigkeitsleitende Verbindung" bedeutet hierin, dass eine Monomerflüssigkeit aus einem Teil der Vorrichtung zu einem anderen Teil der Vorrichtung fließen kann.

Das Septum kann ein gummielastisches Material aufweisen. In einigen Ausführungsformen weist das Septum ein flexibles, selbstdichtendes Polymer auf. Das Polymer kann ein polyhalogeniertes Olefin, ein Silikon, Kautschuk, Butylkautschuk, Ethylen-Propylen-DienKautschuk (EPDM), oder ein ähnliches Elastomer aufweisen. Das Septum ist bevorzugt eingerichtet, nach einem Durchstechen des Septums mit dem Hohldorn die Kartusche flüssigkeitsdicht zu verschließen. Das Septum ist bevorzugt dehnbar und gummielastisch, wie es beispielsweise von Septen in Chemikalienflaschen bekannt ist. Dadurch kann sich das Septum nach einem Durchstich selbst heilen, d.h. selbsttätig abdichten.

Bevorzugt weist das Septum ein biokompatibles Material auf. Das Septum kann eine kreisförmige Form aufweisen. In einer Ausführungsform weist das Septum ein Material auf, das mit einer medizinischen Injektionskanüle gemäß der Norm ISO 7864:2016 mit einer Kraft von weniger als 100 N durchstechbar ist. Das Septum ist bevorzugt flüssigkeitsdicht mit der Kartusche verbunden. In einer Ausführungsform ist das Septum ausgebildet und eingerichtet, nach einem Einstich und anschließender Entfernung des Hohldorns eine Einstichstelle in dem Septum flüssigkeitsdicht zu verschließen. Dies kann durch die Rückstellkraft eines gummielastischen Materials des Septums erfolgen.

Die Kartusche kann ein Führungselement aufweisen, welches ein Einführen des Hohldorns in eine gewünschte Position des Septums erleichtert, z.B. eine Einführungsschräge.

Das Septum kann in einer Halterung gelagert sein. Die Halterung kann lösbar mit der Kartusche verbunden sein. Die Halterung des Septums kann als Kolben ausgestaltet sein. Dieser Kolben kann zur Abgabe von Knochenzementteig aus der Kartusche ausgebildet und eingerichtet sein. Die Halterung für das Septum kann in einem Kartuschenkopf angeordnet sein. Der Kartuschenkopf kann als lösbarer Verschluss der Kartusche ausgebildet sein.

Die Kartusche und das Basisteil sind bevorzugt getrennt voneinander lagerbar, d.h. sie sind als separate Teilelemente ausgestaltet, die lösbar miteinander verbindbar sind. Dadurch kann eine Ermüdung der Rückstellkraft des Septums während der Lagerung verhindert und das selbsttätige Schließen des Septums erleichtert werden. Ein unerwünschter Austritt einer Monomerflüssigkeit aus der Kartusche nach dem Entfernen des Hohldorns aus dem Septum kann verhindert werden. Weiterhin hat die getrennte Lagerung den Vorteil, dass Packmittel mit geringen Abmessungen verwendbar sind.

Die Vorrichtung weist einen Kolben auf. Der Kolben kann innerhalb des Basisteils angeordnet sein. Der Kolben kann eine Durchführung zur Aufnahme des Hohldorns aufweisen. Die Durchführung kann sich von einem Innenraum des Basisteils nach außen erstrecken. Der Kolben kann eingerichtet sein, in einer Grundkonfiguration der Vorrichtung die Spitze des Hohldorns in der Durchführung aufzunehmen. In einer solchen Grundkonfiguration der Vorrichtung wird keine äußere Kraft auf die Vorrichtung ausgeübt. Die Spitze des Hohldorns ist in diesem Zustand bevorzugt innerhalb des Kolbens angeordnet, so dass ein Benutzer vor Verletzung durch Kontakt mit der Spitze geschützt ist. In einer Ausführungsform ist der Kolben eingerichtet, die Spitze des Hohldorns aus der Durchführung freizugeben, wenn die Kartusche auf den Kolben gedrückt wird. Dies ermöglicht es, mit der Kartusche Druck auf den Kolben auszuüben, um den Hohldorn freizugeben und das Septum der Kartusche zu durchstechen. Hierbei kann die Spitze des Hohldorns aus der Durchführung herausragen. Somit können Kartusche und Basisteil verletzungssicher fluidleitend miteinander verbunden werden, um eine Monomerflüssigkeit in die Kartusche aufzunehmen. Der Kolben ist bevorzugt eingerichtet, durch die Kartusche in das Basisteil eingeschoben zu werden, wenn die Kartusche mit dem Basisteil verbunden ist. Durch Ausübung von Druck gegen den Kolben durch die Kartusche ist der Kolben demnach beweglich.

In einigen Ausführungsformen ist die Spitze des Hohldorns durch eine lösbare Abdeckung geschützt.

Erfindungsgemäß ist der Kolben eingerichtet, eine Monomerflüssigkeit aus dem Sammelbereich durch den Hohldorn in die Kartusche zu pumpen. Der Kolben kann hierzu eingerichtet sein, den Druck innerhalb des Sammelbereichs zu erhöhen, so dass der Druck innerhalb des Sammelbereichs den Druck in der Kartusche übersteigt. In einigen Ausführungsformen ist die Vorrichtung ausgebildet und eingerichtet, unabhängig von einer externen Vakuumquelle einen Transfer einer Monomerflüssigkeit in die Kartusche zu ermöglichen.

In einigen Ausführungsformen ist der Kolben beweglich in dem Basisteil angeordnet. Durch Druck auf den Kolben kann der Kolben bevorzugt in Richtung des Sammelbereichs des Basisteils beweglich sein. Der Kolben kann in diesem Fall also in Richtung des Innenraums des Basisteils bzw. in Richtung einer Aufstandsfläche des Basisteils bewegt werden. Durch diese Bewegung kann der Druck im Sammelbereich erhöht werden, um eine Monomerflüssigkeit aus dem Sammelbereich durch den Hohldorn in die Kartusche zu pumpen.

Bevorzugt ist das Bedienelement operativ mit dem Kolben verbunden oder verbindbar. Die Vorrichtung kann insbesondere eingerichtet sein, abhängig von der Anordnung des Bedienelements die Funktion des Kolbens zu steuern. Wie hierin beschrieben, kann die Pumpfunktion in einer ersten Anordnung des Bedienelements deaktiviert sein, während die Pumpfunktion in einer zweiten Anordnung des Bedienelements ermöglicht sein kann.

In einigen Ausführungsformen ist der Kolben ausgebildet und eingerichtet, vollständig in den Sammelbereich eingeschoben zu werden, so dass kein Totvolumen in dem Sammelbereich verbleibt. Hierzu kann eine Seite des Kolbens, die in Richtung des Sammelbereichs ausgerichtet ist, eine Oberflächenform aufweisen, welche komplementär zu einer Oberflächenform des Sammelbereichs ist. Beispielsweise kann der Kolben eine konvexe Frontseite aufweisen, und der Sammelbereich kann eine konkave Oberfläche aufweisen. Der Kolben kann eine Erhebung aufweisen, die komplementär zu einer Vertiefung des Sammelbereichs ist. Dies ermöglicht eine im Wesentlichen vollständige Überführung von Monomerflüssigkeit aus dem Sammelbereich in die Kartusche, da lediglich ein geringfügiges Totvolumen innerhalb des Basisteils (insbesondere innerhalb des Hohldorns) verbleibt, wenn die Monomerflüssigkeit durch die Bewegung des Kolbens aus dem Basisteil in die Kartusche gepumpt wird.

In einigen Ausführungsformen ist die Vorrichtung ausgebildet und eingerichtet, gleichzeitig eine fluidleitende Verbindung zwischen Basisteil und Kartusche herzustellen, und ein Gefäß mit einer Monomerflüssigkeit zu öffnen. Hierbei kann die Kartusche Druck auf den Kolben ausüben, um den Hohldorn aus der Durchführung freizugeben und das Septum zu durchstoßen, wobei gleichzeitig ein Öffnungselement das Gefäß öffnet. Hierzu kann der Kolben operativ mit dem Öffnungselement verbunden sein.

In einigen Ausführungsformen ist die Vorrichtung ausgebildet und eingerichtet, durch eine erste Bewegung des Kolbens ein Gefäß mit einer Monomerflüssigkeit zu öffnen, und durch eine zweite Bewegung des Kolbens eine Monomerflüssigkeit aus dem Sammelbereich in die Kartusche zu überführen, wobei die zweite Bewegung durch den Übergang des Bedienelements von einer ersten Anordnung des Bedienelements in eine zweite Anordnung des Bedienelements ermöglicht ist.

Der Kolben kann eine lösbare Sicherung aufweisen, welche eine unbeabsichtigte Bewegung des Kolbens verhindert, beispielsweise während des Transports oder einer sonstigen Zeit des Nichtgebrauchs der Vorrichtung. Diese Sicherung kann beispielsweise als offene Ringklemme ausgestaltet sein. Hierdurch kann ein unerwünschtes Öffnen eines Gefäßes mit Monomerflüssigkeit in der Vorrichtung vermieden werden.

In einer Ausführungsform weist das Basisteil eine Feder auf, welche zur beweglichen Lagerung des Kolbens innerhalb des Basisteils angeordnet und eingerichtet ist. Hierdurch kann der Kolben in einer Position gehalten werden, in der die Spitze des Hohldorns wie oben beschrieben in der Durchführung des Kolbens aufgenommen und somit verletzungssicher innerhalb des Kolbens gehalten ist. Die Feder kann eine Spiralfeder sein. In einer Ausführungsform umgibt die Feder den Hohldorn in einem Abschnitt des Hohldorns, der sich außerhalb der Durchführung des Kolbens befindet. Die Feder kann zwischen dem Bodenteil und dem Kolben angeordnet sein.

In einer Ausführungsform, in welcher der Hohldorn in einer zylinderförmigen Halterung gelagert ist, wie hierin beschrieben, kann die Feder zwischen dieser Halterung und dem Kolben angeordnet sein. Die Feder kann die Trennung von Basisteil und Kartusche erleichtern. Weiterhin kann die Spitze des Hohldorns durch die Feder selbsttätig zurück in den Hohlraum bewegt werden, sobald keine externe Kraft mehr auf den Kolben einwirkt, z.B. wenn der Benutzer die Kartusche nicht mehr aktiv gegen das Basisteil drückt. Die Feder kann auch eingerichtet sein, den Kolben in einer Position zu halten, in welcher der Verbindungskanal offen ist, d. h. wobei der Verbindungskanal eine fluidleitende Verbindung zwischen dem Behälter und dem Sammelbereich bildet, und der Kolben die Ausgangsöffnung des Verbindungskanals nicht dicht sind verschließt.

In einer Ausführungsform weist das Basisteil einen Verbindungskanal mit einer Ausgangsöffnung auf. Der Verbindungskanal kann eine fluidleitende Verbindung zwischen dem Behälter und dem Sammelbereich bilden. Somit kann eine Monomerflüssigkeit aus dem Behälter über den Verbindungskanal in den Sammelbereich überführt werden. Der Verbindungskanal weist bevorzugt eine Ausgangsöffnung auf, wobei die Ausgangsöffnung bevorzugt an einem Ende des Verbindungskanals angeordnet ist, welches in Richtung des Sammelbereichs weist. Bevorzugt ist der Verbindungskanal ausgebildet und eingerichtet, Monomerflüssigkeit aus dem Behälter durch freien Gravitationsfluss in den Sammelbereich zu überführen. Dies gilt insbesondere für eine Anordnung der Vorrichtung, in welcher das Basisteil auf einer Unterlage aufgestellt ist, so dass das Bodenteil in Richtung der Unterlage ausgerichtet ist, wie hierin beschrieben. "Freier Gravitationsfluss" bedeutet hierin, dass eine Monomerflüssigkeit allein durch die Wirkung der Schwerkraft aus dem Behälter in den Sammelbereich gelangen kann, ohne dass hierzu beispielsweise ein Druckunterschied zwischen dem Behälter und dem Sammelbereich erforderlich ist. Bevorzugt erlauben die hierin beschriebenen Vorrichtungen eine vollständige Überführung der Monomerflüssigkeit aus dem Behälter in den Sammelbereich durch freien Gravitationsfluss innerhalb von 20 Sekunden. Anschließend kann die Monomerflüssigkeit durch Pumpen mithilfe des Kolbens aus dem Sammelbereich in die Kartusche überführt werden, wie hierin beschrieben.

In einer Ausführungsform ist der Kolben ausgebildet und eingerichtet, die Ausgangsöffnung des Verbindungskanals flüssigkeitsdicht zu verschließen, wenn der Kolben in das Basisteil eingeschoben wird. Durch eine Bewegung des Kolbens in Richtung des Sammelbereichs kann eine Seitenfläche des Kolbens in Kontakt mit der Ausgangsöffnung des Verbindungskanals gebracht werden, um die Ausgangsöffnung zu verschließen. In einer solchen Anordnung der Vorrichtung besteht keine fluidleitende Verbindung zwischen dem Behälter und dem Sammelbereich. Der Sammelbereich ist in dieser Konfiguration durch den Kolben von dem Behälter abgedichtet, so dass lediglich eine fluidleitende Verbindung des Sammelbereichs mit dem Hohldorn besteht. Dies ermöglicht es dem Kolben, Monomerflüssigkeit aus dem Sammelbereich durch den Hohldorn zu pumpen, da in dieser Anordnung kein Druckausgleich über den Verbindungskanal mit dem Behälter erfolgen kann.

Die Vorrichtung ist bevorzugt eingerichtet, in einer ersten Anordnung des Bedienelements die Ausgangsöffnung freizugeben, und in einer zweiten Anordnung des Bedienelements die Ausgangsöffnung mithilfe des Kolbens zu verschließen.

Die Ausgangsöffnung kann eine Querschnittsfläche von 50 mm², bevorzugt von mindestens 100 mm² und besonders bevorzugt von größer 100 mm² aufweisen.

Abhängig von der Anordnung des Bedienelements kann die Position des Kolbens oberhalb oder unterhalb der Ausgangsöffnung gehalten sein, entsprechend einer geöffneten bzw. geschlossenen Ausgangsöffnung. Demnach kann die erfindungsgemäße Vorrichtung ausgebildet und eingerichtet sein, in einer hierin beschriebenen ersten Anordnung des Bedienelements Monomerflüssigkeit aus dem Behälter in den Sammelbereich abzugeben, und in einer hierin beschriebenen zweiten Anordnung des Bedienelements Monomerflüssigkeit aus dem Sammelbereich durch den Hohldorn zu pumpen. Mithilfe des Hohldorns kann das Septum der Kartusche durchstechbar sein, um Monomerflüssigkeit aus dem Basisteil in die Kartusche zu überführen.

In einer Ausführungsform weist die Vorrichtung einen Filter auf. Der Filter kann im Basisteil angeordnet sein. Beispielsweise kann der Filter zwischen dem Behälter und dem Hohldorn angeordnet sein. Der Filter kann zwischen dem Behälter und dem Verbindungskanal angeordnet sein. Der Filter ist bevorzugt ausgebildet und eingerichtet, ein Eindringen von Partikeln aus dem Basisteil in die Kartusche zu verhindern. Beispielsweise kann der Filter angeordnet und eingerichtet sein, Glasbruchstücke von einer in dem Basisteil aufgebrochenen Glasampulle zurückhalten. Der Filter kann beispielsweise ein Metalldrahtgeflecht oder ein Netz aus einem Kunststoffgewebe sein. Der Filter kann auch eine mit zweckmäßig dimensionierten Löchern versehene Scheibe aus Metall oder Kunststoff sein. Weiterhin können offenporige Schaumstoffe wie zum Beispiel Porex verwendet werden. Bevorzugt sollte der Filter aus einem Material gefertigt sein, welches durch die in einer aufnehmbaren Glasampulle befindliche Monomerflüssigkeit nicht angegriffen wird.

In einer Ausführungsform ist der Behälter eingerichtet, das Überführen einer Monomerflüssigkeit aus dem Behälter in den Sammelbereich durch freien Gravitationsfluss zu ermöglichen.

In einer Ausführungsform ist der Verbindungskanal in einem Winkel zu dem Bodenteil angeordnet, welcher 20° bis 80°, bevorzugt 21° bis 55°, beträgt. Dieser Winkel ist durch die Innenseite von jeweils Behälter und Bodenteil definiert, wo Verbindungskanal und Bodenteil in Richtung des Bodenteils (d.h. in Richtung der "Unterseite" des Basisteils) an einer senkrechten Seitenwand des Bodenteils ineinander übergehen. Dies entspricht der Stelle, an der bei bestimmungsgemäßem Gebrauch der Vorrichtung die Monomerflüssigkeit aus dem Verbindungskanal in den Sammelbereich fließt. Der Behälter ist bevorzugt gegenüberliegend zum Bodenteil angeordnet, d.h. bei bestimmungsgemäßem Gebrauch der Vorrichtung "nach oben" ausgerichtet, um ein Abfließen der Monomerflüssigkeit in den Sammelbereich zu ermöglichen. Beispielsweise kann der Verbindungskanal zum Bodenteil in ähnlicher Weise wie ein Flügel einer Flügelmutter angeordnet sein. Das Basisteil kann beispielsweise zwei oder mehr Behälter und Verbindungskanäle aufweisen. In einer Ausführungsform umfasst das Basisteil zwei angeordnete Verbindungskanäle, welche jeweils Y-förmig zu dem Bodenteil angeordnet sind. Der Kolben kann hierbei zwischen den Behältern angeordnet sein, d.h. zwischen den beiden Verbindungskanälen, welche die "Y-Arme" bilden.

Der Behälter kann angeordnet sein, so dass sich dieser in einer Konfiguration des Basisteils, in welcher das Basisteil auf einer Unterlage aufgestellt ist, wobei das Bodenteil in Richtung der Unterlage ausgerichtet ist, oberhalb des Sammelbereichs befindet. Insbesondere kann auch die Ausgangsöffnung des Verbindungskanals in einer solchen Konfiguration oberhalb des Sammelbereichs angeordnet sein. Dies erlaubt ein zuverlässiges Abfließen von Monomerflüssigkeit aus dem Behälter in den Sammelbereich. Weiterhin ermöglicht dies eine Anordnung des Kolbens, in welcher die Ausgangsöffnung des Verbindungskanals durch den Kolben abgedichtet ist, und eine Aufnahme von Monomerflüssigkeit in dem Sammelbereich möglich ist, d. h. ausreichend freies Volumen im Sammelbereich zur Verfügung steht, um Monomerflüssigkeit zu beherbergen.

In einer Ausführungsform ist eine Längsachse des Verbindungskanals in einem Winkel zu einer Achse, die senkrecht zu einer Aufstandsfläche des Bodenteils verläuft, angeordnet, welcher 20° bis 80°, bevorzugt 21° bis 55°, beträgt. Bei einem Winkel von 21° bis 55° kann die Ausbildung eines Meniskus durch die Monomerflüssigkeit in besonderem Maße verhindert werden.

In einer Ausführungsform ist die Kartusche lösbar mit dem Basisteil verbindbar. Hierzu können an der Kartusche und am Basisteil entsprechende Verbindungselemente vorgesehen sein, wie z.B. ein Gewinde. Die Verbindungselemente können derart angeordnet sein, dass sie eine Verbindung des Hohldorns mit dem Septum erlauben. Weiterhin kann die Vorrichtung ein Führungselement aufweisen, welche die Verbindung des Basisteils mit der Kartusche in einer gewünschten Orientierung erleichtert. Bevorzugt sind Basisteil und Kartusche derart miteinander verbindbar, dass der Kolben und das Septum bündig miteinander abschließen. Hierzu kann beispielsweise das Basisteil einen überstehenden Bereich aufweisen, der ausgestaltet und eingerichtet ist, in die Kartusche einzugreifen.

In einer Ausführungsform weist das Basisteil ein Leitungselement auf, wobei das Leitungselement eingerichtet ist, eine fluidleitende Verbindung zwischen dem Sammelbereich und dem Hohldorn herzustellen. Das Leitungselement ermöglicht eine Überführung von Monomerflüssigkeit aus dem Sammelbereich in den Hohldorn, bevorzugt eine Überführung durch freien Gravitationsfluss. Das Leitungselement kann beispielsweise als Schlauch, Rohr oder Durchführung ausgestaltet sein. In einer Ausführungsform, in welcher der Hohldorn in einer zylinderförmigen Halterung gelagert ist, wie hierin beschrieben, kann das Leitungselement als Durchführung in der Halterung ausgestaltet sein.

In einer Ausführungsform ist die Vorrichtung ausgestaltet und eingerichtet, eine Monomerflüssigkeit aus dem Basisteil in die Kartusche zu überführen, während das Basisteil auf einer Unterlage aufgestellt ist, so dass das Bodenteil in Richtung der Unterlage ausgerichtet ist. Hierbei weisen die Behälter und die Kartusche bevorzugt nach "oben", d.h. in entgegengesetzter Richtung zur Unterlage, und entgegen der Schwerkraftrichtung. Die Monomerflüssigkeit kann daher durch die Wirkung der Schwerkraft aus dem Behälter in den Sammelbereich fließen.

In einigen Ausführungsformen weist die Kartusche ein PMMA-Knochenzementpulver auf. Das PMMA-Knochenzementpulver ist bevorzugt in einem Innenraum der Kartusche angeordnet.

In einigen Ausführungsformen weist der Behälter eine Monomerflüssigkeit auf. Die Monomerflüssigkeit ist bevorzugt in einem Innenraum des Behälters angeordnet. Die Monomerflüssigkeit kann in einem Gefäß, wie z.B. einer Glasampulle oder in einem versiegelten Folienbeutel, innerhalb des Behälters angeordnet sein.

In einigen Ausführungsformen weist der Behälter weiterhin einen für eine Monomerflüssigkeit durchlässigen Filter auf. Der Filter ist bevorzugt angeordnet und eingerichtet, ein Eindringen von Glassplittern aus dem Behälter in die Kartusche zu verhindern. Der Filter kann beispielsweise zwischen dem Träger und dem Verbindungskanal angeordnet sein.

Die Vorrichtung kann zur Aufnahme einer Glasampulle mit Monomerflüssigkeit eingerichtet sein. Die Vorrichtung kann weiterhin zum Aufbrechen einer Glasampulle mit Monomerflüssigkeit eingerichtet sein. Die Glasampulle kann insbesondere innerhalb des Behälters aufnehmbar und aufbrechbar sein. Der Behälter kann hierzu einen Träger, z.B. eine Aufnahme für eine Glasampulle aufweisen. Der Behälter kann ein Mittel zum Aufbrechen einer Glasampulle aufweisen. Beispielsweise kann der Behälter einen Vorsprung aufweisen, der zum Abbrechen des Kopfs einer Glasampulle angeordnet und eingerichtet ist. Der Behälter kann einen Auffangbereich für Glasbruch aufweisen. Der Filter kann zwischen diesem Auffangbereich und dem Verbindungskanal angeordnet sein.

Der Filter kann eingerichtet sein, Bruchstücke einer Glasampulle in dem Behälter zurückzuhalten. Hierdurch kann vermieden werden, dass Glassplitter in die Kartusche gelangen. Hierdurch kann eine Verunreinigung von Knochenzementteig mit Glassplittern vermieden werden.

Die hierin beschriebenen Filter für Monomerflüssigkeit können entweder im Behälter oder an einer anderen Position des Basisteils angeordnet sein, oder beides. Beispielsweise kann der Behälter einen ersten Filter aufweisen, und das Leitungselement, welches den Sammelbereich mit dem Hohldorn verbindet, kann einen zweiten Filter aufweisen. Der zweite Filter kann ein kleineres Ausschlussvolumens als der erste Filter aufweisen.

In einigen Ausführungsformen weist die Vorrichtung einen Mischstab auf. Der Mischstab ist bevorzugt zum Mischen von Monomerflüssigkeit und PMMA-Knochenzementpulver eingerichtet. Der Mischstab kann Flügel aufweisen. Die Flügel können eine homogene Mischung von Monomerflüssigkeit und PMMA-Knochenzementpulver erleichtern. Die Flügel sind bevorzugt an einem Ende des Mischstabs angeordnet, welches innerhalb der Kartusche angeordnet ist. Die Flügel können an einer Scheibe des Mischstabs angeordnet sein. Der Mischstab kann einen, zwei oder mehr als zwei Flügel aufweisen. Der Mischstab kann an einem Ende des Mischstabs, welches bevorzugt außerhalb der Kartusche angeordnet ist, einen Griff aufweisen. Der Mischstab ist bevorzugt beweglich in der Kartusche angeordnet.

Der Mischstab kann ein Austragsrohr aufweisen. Hierbei kann das Austragsrohr zur Abgabe eines in der Vorrichtung gemischten Knochenzementteigs (d.h. eines Knochenzementteigs, der aus Monomerflüssigkeit und PMMA-Knochenzementpulver gemischt wurde) ausgebildet und eingerichtet sein. Bevorzugt ist das Austragsrohr verschließbar. Hierdurch kann verhindert werden, dass Knochenzementteig während des Mischens unerwünscht aus der Kartusche austritt. Das Austragsrohr kann als Hohlraum innerhalb des Mischstabs ausgebildet sein. In einigen Ausführungsformen ist das Austragsrohr durch einen lösbaren Kern verschließbar. Demnach kann der Mischstab einen lösbaren, stabförmigen Kern aufweisen, beispielsweise einen Metallkern. Der Kern kann auch die Festigkeit des Mischstabs verbessern.

Der Mischstab kann eine Sollbruchstelle aufweisen, um den Mischstab nach dem Mischvorgang abzubrechen. Die Sollbruchstelle ist bevorzugt außerhalb der Kartusche angeordnet, so dass der Mischstab ohne ein Öffnen der Kartusche von außen abgebrochen werden kann.

Die Kartusche kann einen Anschluss für eine Vakuumquelle, z.B. eine Pumpe, aufweisen. Dies ermöglicht ein Mischen von Monomerflüssigkeit und PMMA-Knochenzementpulver unter Vakuumbedingungen in der Kartusche.

Weiterhin kann das Basisteil eine Belüftungsöffnung aufweisen. Die Belüftungsöffnung kann in dem Behälter angeordnet sein. Die Belüftungsöffnung kann bevorzugt derart in dem Basisteil angeordnet sein, um einen Druckausgleich zu erlauben, während der Kolben die Ausgangsöffnung des Verbindungskanals freihält, d.h. nicht verschließt. Vorzugsweise ist die Belüftungsöffnung derart in dem Basisteil angeordnet, dass ein Kontakt der Belüftungsöffnung mit Monomerflüssigkeit bei bestimmungsgemäßem Gebrauch der Vorrichtung vermieden wird. Die Belüftungsöffnung kann wahlweise als einfache Öffnung oder als Ventil ausgestaltet sein. Die Öffnung kann an der Innenseite durch ein gasdurchlässiges poröses Material abgedeckt sein, so dass das Eindringen von Partikeln von außen in den Behälter vermieden werden kann, und gleichzeitig ein Luftaustausch durch das poröse Material möglich ist. Das Ventil kann beispielsweise ausgestaltet sein, Luft nur in Richtung des Innenraums des Behälters passieren zu lassen. Hierdurch kann der Transfer der Monomerflüssigkeit in den Sammelbereich verbessert werden. Weiterhin kann ein unidirektionales Ventil das unerwünschte Austreten von Monomerflüssigkeit aus der Vorrichtung verhindern.

Ein weiterer Aspekt betrifft ein Verfahren zur Herstellung eines Knochenzementteigs, wobei das Verfahren die nachfolgenden Schritte aufweist,
Bereitstellen einer Vorrichtung nach einer der hierin beschriebenen Ausführungsformen; gegebenenfalls Einbringen eines Gefäßes mit einer Monomerflüssigkeit in den Behälter; gegebenenfalls Einbringen eines PMMA-Knochenzementpulvers in die Kartusche; Inkontaktbringen der Kartusche mit dem Basisteil;
Einstechen des Hohldorns durch das Septum, sodass eine fluidleitende Verbindung zwischen dem Sammelbereich und der Kartusche gebildet wird;
gegebenenfalls Positionieren des Bedienelements in eine erste Anordnung, welche das Öffnen des Gefäßes erlaubt;
Öffnen des Gefäßes mithilfe des Bedienelements, wobei das Bedienelement eine Kraft auf das Gefäß ausübt, um dieses zu öffnen, und eine Monomerflüssigkeit daraus freizusetzen;
gegebenenfalls Anordnen der Vorrichtung, so dass die Monomerflüssigkeit aus dem Behälter durch freien Gravitationsfluss in den Sammelbereich überführt wird;
Positionieren des Bedienelements in eine zweite Anordnung, welche einen Pumpvorgang des Kolbens erlaubt;
Drücken der Kartusche gegen den Kolben, sodass Monomerflüssigkeit aus dem Behälter in die Kartusche gepumpt wird;
Mischen der Monomerflüssigkeit mit dem PMMA-Knochenzementpulver in der Kartusche und dadurch Erhalten eines Knochenzementteigs.

Ein weiterer Aspekt betrifft ein Verfahren zur Herstellung eines Knochenzementteigs, wobei das Verfahren die nachfolgenden Schritte aufweist,
Bereitstellen einer Vorrichtung, welche ein Basisteil mit einem Gefäß mit Monomerflüssigkeit, einem beweglichen Kolben mit einer Durchführung, und einem Hohldorn, und eine Kartusche mit einem Septum und einem PMMA-Knochenzementpulver aufweist, wobei die Vorrichtung weiterhin ein Bedienelement aufweist;
gegebenenfalls Positionieren des Bedienelements in eine erste Anordnung, welche das Öffnen des Gefäßes erlaubt;
Öffnen des Gefäßes mithilfe des Bedienelements, wobei das Bedienelement eine Kraft auf das Gefäß ausübt, um dieses zu öffnen, und eine Monomerflüssigkeit daraus freizusetzen;
Einstechen des Hohldorns durch das Septum, sodass eine fluidleitende Verbindung zwischen dem Sammelbereich und der Kartusche gebildet wird;
Positionieren des Bedienelements in eine zweite Anordnung, welche einen Pumpvorgang des Kolbens erlaubt;
Drücken der Kartusche gegen den Kolben, sodass Monomerflüssigkeit aus dem Behälter in die Kartusche gepumpt wird;
Mischen der Monomerflüssigkeit mit dem PMMA-Knochenzementpulver in der Kartusche und dadurch Erhalten eines Knochenzementteigs.

Ein weiterer Aspekt betrifft die Verwendung einer hierin beschriebenen Vorrichtung oder des vorangehend beschriebenen Verfahrens zur Herstellung von Knochenzementteig, insbesondere PMMA-Knochenzementteig. Hierbei kann eine Monomerflüssigkeit mit einem PMMA-Knochenzementpulver innerhalb der Vorrichtung gemischt werden, um einen Knochenzementteig zu bilden. Ein solcher Knochenzementteig kann aus der Vorrichtung abgegeben werden und an einem Zielort zu PMMA-Knochenzement aushärten.

### BEISPIELE

Die Erfindung wird nachfolgend anhand von Beispielen weiter verdeutlicht, die jedoch nicht als einschränkend zu verstehen sind. Dem Fachmann wird ersichtlich sein, dass anstelle der hier beschriebenen Merkmale andere äquivalente Mittel in ähnlicher Weise verwendet werden können.

### FIGUREN

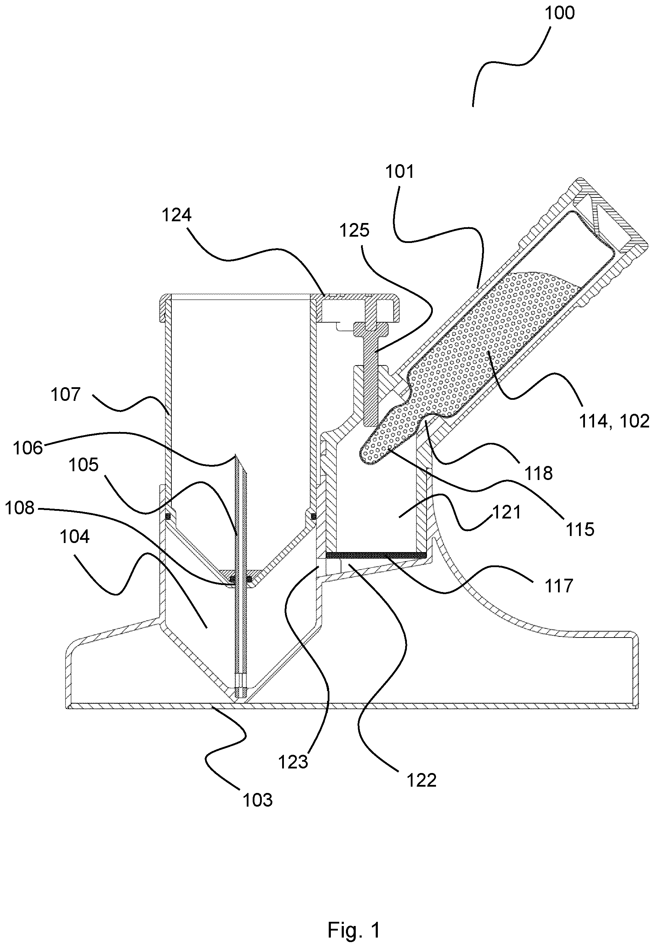

**Figur 1** zeigt den Querschnitt des Basisteils einer erfindungsgemäßen Vorrichtung. Das Basisteil **100** weist einen Behälter **101** zur Aufnahme einer Monomerflüssigkeit auf. In dem Behälter **101** ist ein Träger **118** angeordnet, der eine Glasampulle **114** mit Monomerflüssigkeit **102** hält. Das Basisteil weist weiterhin ein Bodenteil **103** auf, welches eine ebene Aufstandsfläche zum Aufstellen des Basisteils auf eine Unterlage aufweist. An dem Bodenteil ist ein Sammelbereich **104** zur Aufnahme einer Monomerflüssigkeit angeordnet. Der Sammelbereich weist eine Vertiefung auf, wobei am tiefsten Punkt dieser Vertiefung ein Hohldorn **105** angeordnet ist. Der Hohldorn ist flüssigkeitsleitend mit dem Sammelbereich verbunden, sodass Monomerflüssigkeit aus dem Sammelbereich in den Hohldorn gelangen kann. Der Behälter weist weiterhin einen Auffangbereich **121** auf, um Glasbruchstücke wie z.B. einen abgebrochenen Ampullenkopf **115** aufzunehmen und darin zurückzuhalten. Der Behälter ist über einen Verbindungskanal **122** mit dem Sammelbereich **104** flüssigkeitsleitend verbunden. Somit kann eine Monomerflüssigkeit **102** aus dem Behälter **101** in den Sammelbereich **104** gelangen. Zwischen dem Auffangbereich **121** und dem Verbindungskanal **122** ist ein Filter **117** angeordnet. Mithilfe des Filters **117** können Glasbruchstücke in dem Auffangbereich **121** zurückgehalten werden. Wenn die Vorrichtung mit dem Bodenteil **103** auf eine Unterlage aufgestellt ist, kann Monomerflüssigkeit aus dem Behälter durch Gravitationsfluss in den Sammelbereich gelangen. Somit ist kein Druckunterschied zum Transfer der Monomerflüssigkeit aus dem Behälter in den Sammelbereich erforderlich. Der Verbindungskanal **122** weist an einem Ende des Verbindungskanals, welches sich in Richtung des Sammelbereichs **104** erstreckt, eine Ausgangsöffnung **123** auf. Die Ausgangsöffnung **123** ist oberhalb des Sammelbereichs **104** angeordnet. Das Basisteil **100** weist weiterhin einen Kolben **107** mit einer Durchführung **108** auf. Der Hohldorn **105** ist in die Durchführung **108** aufnehmbar. Der Hohldorn **105** kann eine flüssigkeitsleitende Verbindung zwischen dem Sammelbereich **104** und einer Kartusche **200** herstellen. Der Hohldorn **105** ist in der hier gezeigten Anordnung durch die Durchführung **108** hindurchgeführt. Der Hohldorn **105** weist eine Spitze **106** auf. Das Basisteil **100** weist weiterhin ein Bedienelement **124** auf, welches beweglich an dem Basisteil **100** angeordnet ist. Das Bedienelement **124** ist hier in einer ersten Anordnung gezeigt, in welcher das Bedienelement **124** operativ mit einem Öffnungselement **125** verbunden ist. Das Bedienelement **124** greift in das Öffnungselement **125** ein, sodass das Öffnungselement **125** in das Basisteil **100** abschnittsweise eingeschoben werden kann, wenn ein Benutzer eine Kraft auf das Bedienelement **124** ausübt, welche auf das Öffnungselement **125** übertragbar ist. Das Öffnungselement **125** ist eingerichtet, ein in dem Behälter aufgenommenes Gefäß **114** mit Monomerflüssigkeit zu öffnen. In der hier dargestellten Ausführungsform ist das Öffnungselement **125** als Stift ausgebildet, welcher gegen den Kopf **115** einer Glasampulle **114** gedrückt werden kann, um den Kopf **115** von der Glasampulle **114** abzubrechen und die darin enthaltene Monomerflüssigkeit **102** in das Basisteil **100** freizusetzen.

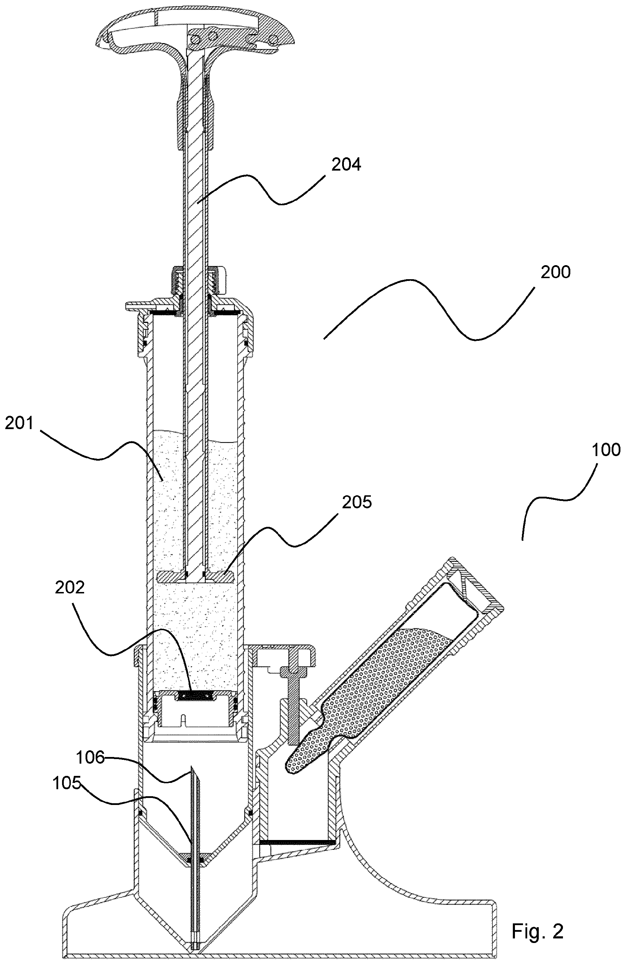

**Figur 2** zeigt den Querschnitt einer erfindungsgemäßen Vorrichtung, wobei ein Basisteil **100** mit einer Kartusche **200** in Kontakt gebracht ist. Die Kartusche **200** weist ein Septum **202** aus einem gummielastischen Material auf, welches einen Innenraum der Kartusche, in welchem ein PMMA-Knochenzementpulver **201** enthalten ist, nach außen begrenzt. Die Kartusche weist weiterhin einen Mischstab **204** mit einer Mischscheibe **205** auf.

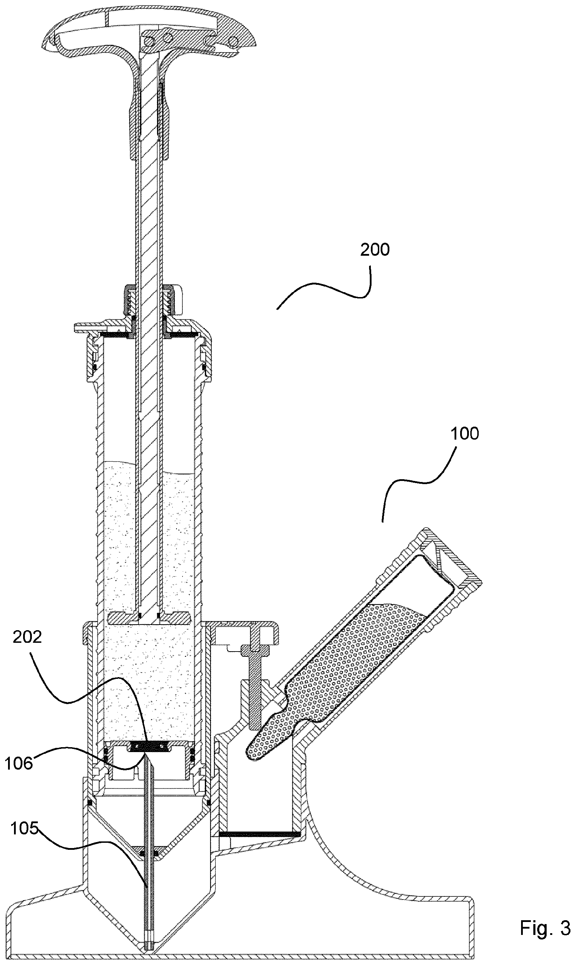

**Figur 3** zeigt den Querschnitt einer erfindungsgemäßen Vorrichtung, wobei eine Kartusche **200** auf ein Basisteil **100** aufgesetzt ist, um ein Septum **202** der Kartusche **200** mit einem Hohldorn **105** des Basisteils zu durchstechen. Die Kartusche **200** ist lösbar mit dem Basisteil **100** verbindbar, sodass die Spitze **106** des Hohldorns **105** das Septum **202** durchdringen kann, und hierdurch eine flüssigkeitsleitende Verbindung zwischen dem Sammelbereich **104** und einem Innenraum der Kartusche **200** herstellbar ist.

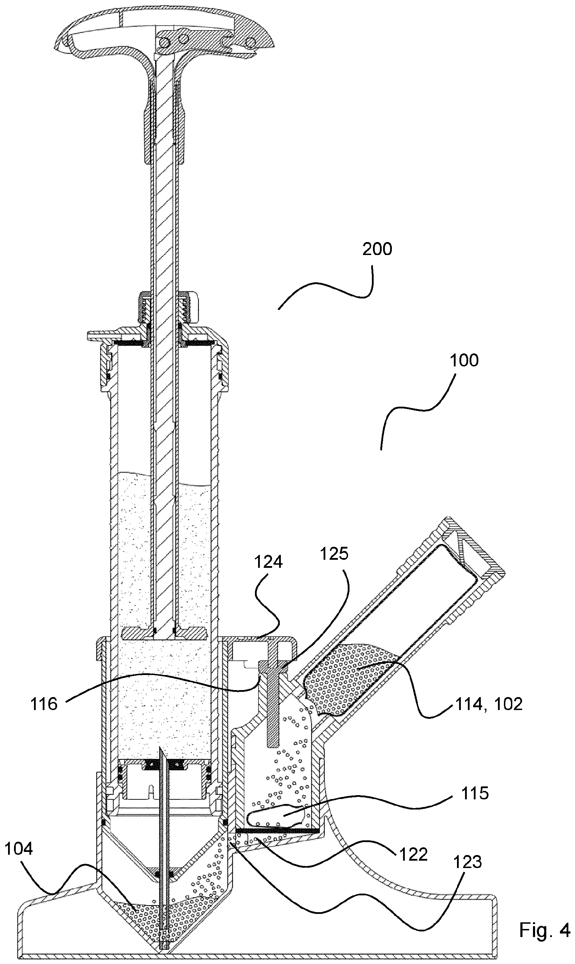

**Figur 4** zeigt den Querschnitt einer erfindungsgemäßen Vorrichtung, wobei das Bedienelement **124** eine Kraft auf das Öffnungselement **125** ausübt, sodass eine Glasampulle **114** in dem Behälter **101** geöffnet wird. Das Öffnungselement **125** ist hier als Stift ausgestaltet, welcher gegen den Kopf **115** einer Glasampulle **114** gedrückt werden kann. Hierdurch wird die Glasampulle **114** geöffnet, um Monomerflüssigkeit **102** aus der Glasampulle freizusetzen.
   Die Monomerflüssigkeit **102** kann in einen Sammelbereich **104** des Basisteils **100** fließen. Dabei fließt die Monomerflüssigkeit **102** aus der Glasampulle **114** über einen Verbindungskanal **122** durch eine Ausgangsöffnung **123** des Verbindungskanals **122** in den Sammelbereich **104.** Ein Anschlag **116** steht in Eingriff mit dem Öffnungselement **125,** welches wiederum in Eingriff mit dem Bedienelement **124** steht. Dies verhindert ein vollständiges Einschieben des Kolbens **107** in den Sammelbereich **104,** sodass die Pumpfunktion des Kolbens **107** in dieser ersten Anordnung des Bedienelements **124** blockiert ist.

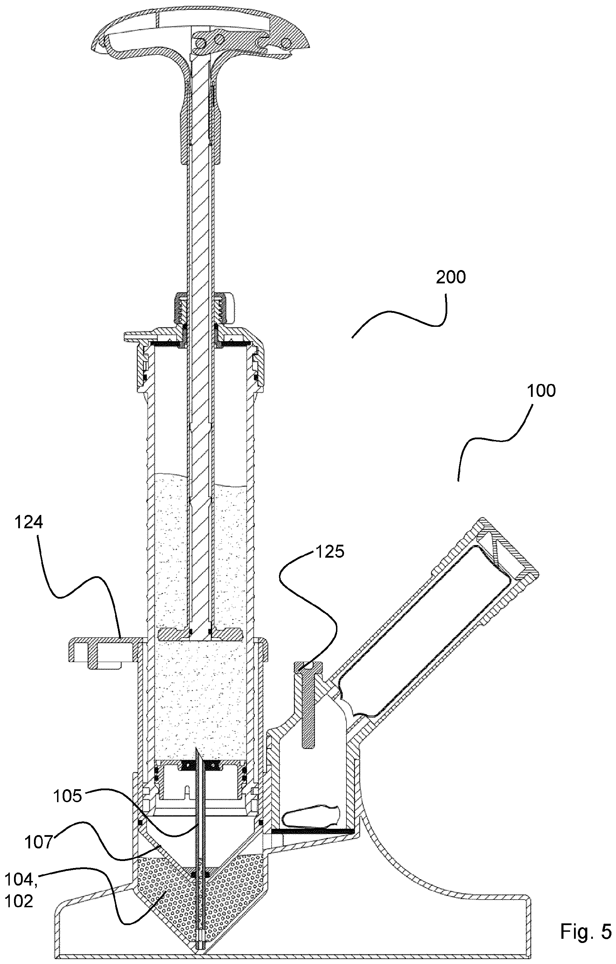

**Figur 5** zeigt den Querschnitt einer erfindungsgemäßen Vorrichtung, wobei eine Kartusche **200** auf ein Basisteil **100** aufgesetzt ist, wobei das Septum **202** der Kartusche **200** mit einem Hohldorn **105** des Basisteils durchstochen ist. Das Bedienelement **124** ist in der hier gezeigten zweiten Anordnung des Bedienelements seitlich verschwenkt, sodass es nicht mehr in Eingriff mit dem Öffnungselement **125** steht. In dieser zweiten Anordnung des Bedienelements ist die Pumpfunktion des Kolbens **107** ausführbar. Das Bedienelement **124** kann eine Kraft auf den Kolben **107** ausüben, um diese vollständig in den Sammelbereich **104** zu bewegen. Hierdurch kann Monomerflüssigkeit **102** aus dem Sammelbereich **104** durch den Hohldorn **105** in die Kartusche **200** gepumpt werden.

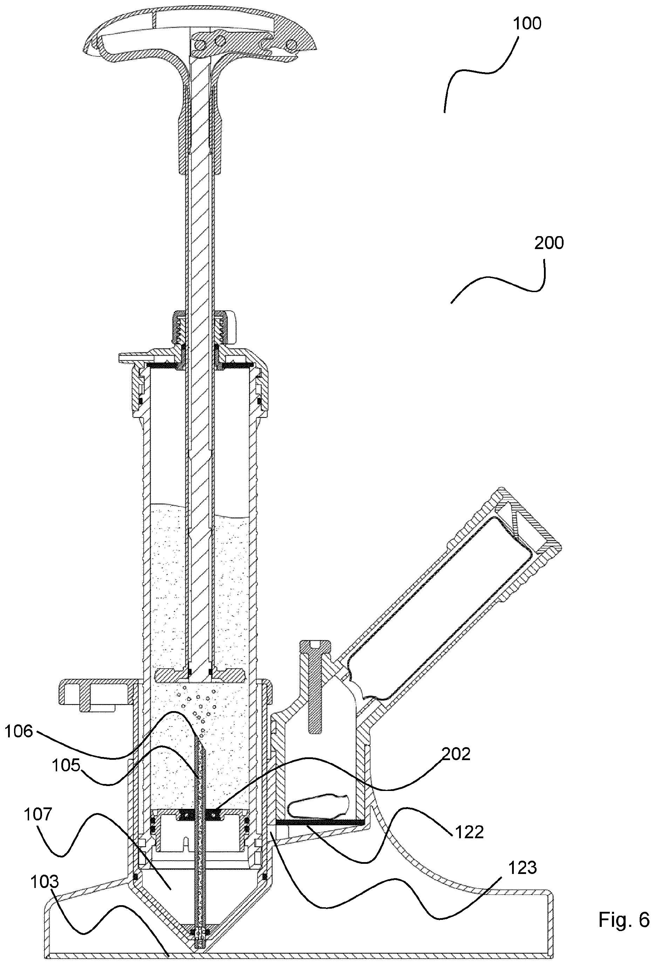

**Figur 6** zeigt den Querschnitt einer erfindungsgemäßen Vorrichtung, wobei ein Kolben **107** in das Basisteil **100** eingeschoben ist, um eine Ausgangsöffnung **123** eines Verbindungskanals **122** flüssigkeitsdicht zu verschließen. Eine Seite des Kolbens schließt dabei mit der Ausgangsöffnung **123** ab, um die Ausgangsöffnung zu verschließen. Die Spitze **106** des Hohldorns durchdringt das Septum **202,** sodass eine flüssigkeitsleitende Verbindung zwischen dem Sammelbereich **104** und dem Innenraum der Kartusche **200** hergestellt wird. Der Kolben **107** übt Druck auf den Sammelbereich **104** aus, um Monomerflüssigkeit **102** aus dem Sammelbereich **104** durch den Hohldorn **105** in die Kartusche **200** zu pumpen.
   Der Kolben **107** ist eingerichtet, eine Monomerflüssigkeit aus dem Sammelbereich durch den Hohldorn **105** in die Kartusche **200** zu pumpen. Der Kolben **107** ist beweglich in dem Basisteil **100** angeordnet. Der Kolben **107** kann in Richtung des Sammelbereichs **104** bzw. in Richtung des Bodenteils **103** in das Basisteil **100** eingeschoben werden, und hierdurch Druck auf den Sammelbereich **104** ausüben, sodass eine Monomerflüssigkeit durch den Hohldorn **105** aus dem Sammelbereich **104** in die Kartusche **200** gelangen kann.

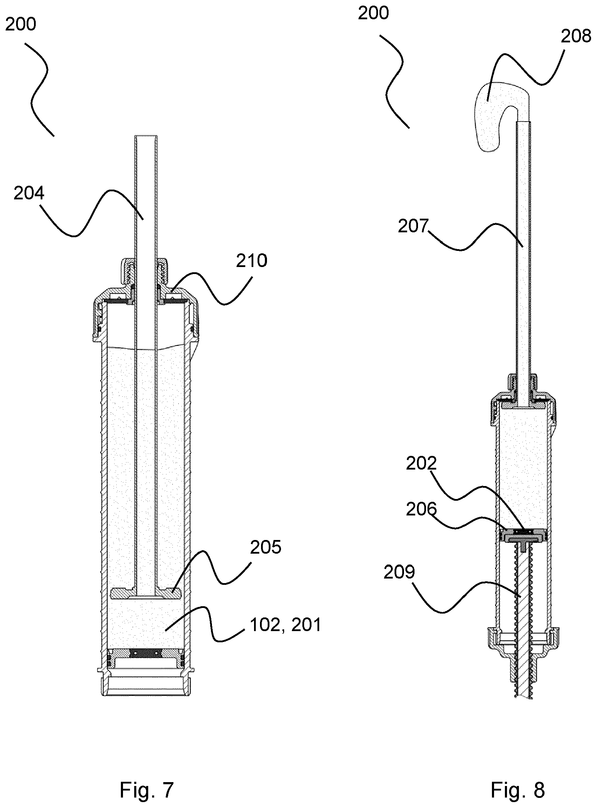

**Figur 7** zeigt einen Querschnitt einer Kartusche 200 mit einem Mischstab **204** zum Mischen einer Monomerflüssigkeit **102** mit einem PMMA-Knochenzementpulver **201** in einem Innenraum der Kartusche **200.** Der Mischstab **204** ist durch einen Kartuschenkopf **210** hindurchgeführt. Der Mischstab **204** ist beweglich in der Kartusche **200** angeordnet und weist eine Mischscheibe **205** auf, welche an einem Ende des Mischstabs **204** innerhalb der Kartusche **200** angeordnet ist, um den Mischvorgang von Monomerflüssigkeit und PMMA-Knochenzementpulver in der Kartusche **200** zu verbessern. Durch Mischen von Monomerflüssigkeit und PMMA-Knochenzementpulver kann ein Knochenzementteig in der Kartusche hergestellt werden.
**Figur 8** zeigt einen Querschnitt einer Kartusche **200** mit einer Gewindestange **209** und einem Austragsrohr **207** zur Abgabe von Knochenzementteig **208** aus der Kartusche **200.** Die Gewindestange **209** ist eingerichtet, eine Halterung **206,** welche das Septum **202** trägt, innerhalb der Kartusche zu bewegen, um Knochenzementteig **208** aus der Kartusche **200** über das Austragsrohr **207** nach außen abzugeben.

### BEZUGSZEICHENLISTE

- 100: Basisteil
- 101: Behälter
- 102: Monomerflüssigkeit
- 103: Bodenteil
- 104: Sammelbereich
- 105: Hohldorn
- 106: Spitze
- 107: Kolben
- 108: Durchführung
- 111: Leitungselement
- 114: Gefäß
- 115: Ampullenkopf
- 116: Anschlag
- 117: Filter
- 118: Träger
- 119: Vorsprung
- 121: Auffangbereich
- 122: Verbindungskanal
- 123: Ausgangsöffnung
- 124: Bedienelement
- 125: Öffnungselement
- 200: Kartusche
- 201: PMMA-Knochenzementpulver
- 202: Septum
- 204: Mischstab
- 205: Mischscheibe
- 206: Halterung für Septum
- 207: Austragsrohr
- 208: Knochenzementteig
- 209: Gewindestange
- 210: Kartuschenkopf

## Patentansprüche

1. Vorrichtung zur Herstellung von Knochenzement, aufweisend
ein Basisteil (100), welches einen Behälter (101) mit einem Träger (118) zur Aufnahme eines Gefäßes mit Monomerflüssigkeit, ein Bodenteil (103) mit einem Sammelbereich (104), einen Hohldorn (105) mit einer Spitze (106), und einen Kolben (107) mit einer Durchführung (108) zur Aufnahme des Hohldorns (105) aufweist, und
eine Kartusche (200) zur Aufnahme eines PMMA-Knochenzementpulvers (201), wobei die Kartusche ein Septum (202) aufweist, welches die Kartusche nach außen begrenzt;
wobei die Vorrichtung weiterhin ein Bedienelement (124) aufweist, und wobei das Bedienelement (124) eingerichtet ist, ein Gefäß mit Monomerflüssigkeit zu öffnen und nachfolgend eine aus dem Gefäß freigesetzte Monomerflüssigkeit durch Bewegung des Kolbens aus dem Sammelbereich durch den Hohldorn in die Kartusche zu pumpen.

2. Vorrichtung gemäß Anspruch 1, wobei das Bedienelement (124) beweglich an dem Basisteil angeordnet ist.

3. Vorrichtung gemäß einem der vorangehenden Ansprüche, wobei das Bedienelement (124) entlang einer Achse verschiebbar und bevorzugt um diese Achse schwenkbar ist.

4. Vorrichtung gemäß einem der vorangehenden Ansprüche, wobei das Bedienelement (124) in einer ersten Anordnung eingerichtet ist, eine Kraft auf ein Gefäß mit Monomerflüssigkeit auszuüben, und dabei ein vollständiges Einschieben des Kolbens (107) in den Sammelbereich (104) zu verhindern.

5. Vorrichtung gemäß einem der vorangehenden Ansprüche, wobei das Bedienelement (124) in einer zweiten Anordnung eingerichtet ist, ein Einschieben des Kolbens (107) in den Sammelbereich (104) zu ermöglichen, um eine Monomerflüssigkeit aus dem Sammelbereich (104) durch den Hohldorn (105) in die Kartusche (200) zu pumpen.

6. Vorrichtung gemäß einem der vorangehenden Ansprüche, weiterhin aufweisend ein Öffnungselement (125), wobei das Bedienelement (124) operativ mit dem Öffnungselement (125) verbindbar oder verbunden ist, wobei das Öffnungselement (125) zum Öffnen eines Gefäßes mit Monomerflüssigkeit ausgebildet und eingerichtet ist.

7. Vorrichtung gemäß einem der vorangehenden Ansprüche, wobei das Bedienelement (124) operativ mit dem Kolben (107) verbindbar oder verbunden ist.

8. Vorrichtung gemäß einem der vorangehenden Ansprüche, wobei das Bedienelement (124) ein Drehgelenk aufweist.

9. Vorrichtung gemäß einem der vorangehenden Ansprüche, wobei die Vorrichtung ausgebildet und eingerichtet ist, mittels Durchstechen des Septums (202) mit dem Hohldorn (105) eine fluidleitende Verbindung zwischen dem Basisteil (100) und der Kartusche (200) zu bilden, um eine Monomerflüssigkeit aus dem Sammelbereich (104) in die Kartusche (200) zu überführen.

10. Vorrichtung gemäß einem der vorangehenden Ansprüche, wobei die Kartusche (200) lösbar mit dem Basisteil (100) verbindbar ist.

11. Vorrichtung gemäß einem der vorangehenden Ansprüche, wobei der Kolben (107) durch Einschieben der Kartusche (200) in das Basisteil (100) beweglich ist.

12. Vorrichtung gemäß einem der vorangehenden Ansprüche, wobei das Basisteil (100) einen Anschlag (116) aufweist, um die Bewegung des Kolbens (107) in dem Basisteil (100) zu begrenzen, wobei das Bedienelement (124) bevorzugt eingerichtet ist, in den Anschlag (116) einzugreifen.

13. Vorrichtung gemäß einem der vorangehenden Ansprüche, wobei das Basisteil (100) einen Verbindungskanal (122) mit einer Ausgangsöffnung (123) aufweist, wobei der Verbindungskanal (122) eine fluidleitende Verbindung zwischen dem Behälter (101) und dem Sammelbereich (104) bildet.

14. Vorrichtung gemäß Anspruch 13, wobei der Kolben (107) ausgebildet und eingerichtet ist, die Ausgangsöffnung (123) des Verbindungskanals (122) flüssigkeitsdicht zu verschließen, wenn der Kolben (107) in das Basisteil (100) eingeschoben wird.

15. Vorrichtung gemäß einem der vorangehenden Ansprüche, wobei die Vorrichtung ausgestaltet und eingerichtet ist, eine Monomerflüssigkeit aus dem Basisteil (100) in die Kartusche (200) zu überführen, während das Basisteil (100) auf einer Unterlage aufgestellt ist, so dass das Bodenteil (103) in Richtung der Unterlage ausgerichtet ist.
